(19) **Europäisches Patentamt**
**European Patent Office**
**Office européen des brevets**

(11) **EP 4 687 144 A1**

(12) **EUROPEAN PATENT APPLICATION**

(43) Date of publication:
**04.02.2026 Bulletin 2026/06**

(21) Application number: **25184363.7**

(22) Date of filing: **23.06.2025**

(51) International Patent Classification (IPC):
**G16C 10/00** $^{(2019.01)}$

(52) Cooperative Patent Classification (CPC):
**G16C 10/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **31.07.2024 IN 202421058105**

(71) Applicant: **Tata Consultancy Services Limited Maharashtra (IN)**

(72) Inventors:
- **RAJAN, Nirmal Mammavalappil**
  **560066 Bengaluru, Karnataka (IN)**
- **KHANDELWAL, Ankit**
  **560066 Bengaluru, Karnataka (IN)**
- **NAMBIAR, Manoj Karunakaran**
  **400021 Mumbai, Maharashtra (IN)**
- **YAMIJALA, Siva Rama Krishna Chaitanya Sharma**
  **600036 Chennai, Tamil Nadu (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD AND SYSTEM FOR SELECTION OF MOLECULAR ACTIVE SPACES THROUGH ELECTRON CORRELATION IDENTIFICATON**

(57) This disclosure relates generally to a selection of molecular active spaces through electron correlation identification. Simulation of complex chemical entities require a lot of computational resources. State-of-art methods suggest to focus on most relevant molecular orbitals that forms an active space. The active space identification mostly rely on chemical intuition and the knowledge of domain experts. These intuitive methods are unreliable for complex systems. The present method discloses selecting correlated molecular active spaces in a chemical entity by generating a set of molecular orbitals (MOs) for a given chemical entity. The active space is identified as a sub-set of a set of relevant MOs. An approximate ground state wavefunction specified in terms of the set of MOs is calculated. A correlation factor is computed for each active space, and it utilized to identify a sub-set of active spaces by segregating the plurality of active spaces based on the correlation factor.

FIG. 3

**Description**

CROSS-REFERENCE TO RELATED APPLICATIONS AND PRIORITY

The present application claims priority from Indian provisional patent application no. 202421058105, filed on July 31, 2024. The entire contents of the aforementioned application are incorporated herein by reference.

TECHNICAL FIELD

The disclosure herein generally relates to computational chemistry, and, more particularly, to selection of correlated molecular active spaces to perform focused quantum chemistry calculations.

BACKGROUND

It is a well-known fact that as the size of a chemical entity under study increases, the computational cost associated with its simulation on a classical computer using exact quantum chemistry approaches grow exponentially. Hence, while simulating complex chemical entities, including all the molecular orbitals of the chemical entity requires a lot of computational resources. By focusing on the computational resources on the most relevant molecular orbitals and electrons, an active space approach reduces the overall complexity and the computational cost. It allows treatment of the chemically important part of the system at a high level of theory, whereas the rest of the system can be modeled at a lower level of theory, making simulations more practical and feasible. Current approaches of choosing the molecular active spaces mostly rely on chemical intuition and the knowledge/experience of the domain experts. Intuitive methods are unreliable for complex systems. One of the widely adopted choices is selecting the highest occupied molecular orbitals (HOMO) and lowest unoccupied molecular orbitals (LUMO) where electronic excitations are found to be highly probable in most cases. However, this is not always true and even for a simple molecule like water in minimal basis sets, the HOMO is found to be less contributing to electron correlation near the equilibrium geometry. Another approach is to consider a large set of possible active spaces and perform coupled cluster singles and doubles (CCSD) or complete active space configuration interaction (CASCI) calculations in each active space to determine the ones recovering maximum correlation energies. As expected, this is highly time-consuming and resource-intensive as methods like CCSD exhibit fourth-order scaling with the system size. Identification of molecular active spaces is necessary to streamline the quantum chemistry calculations associated with large chemical entities.

SUMMARY

Embodiments of the present disclosure present technological improvements as solutions to one or more of the above-mentioned technical problems recognized by the inventors in conventional systems. For example, in one embodiment, a method of selecting correlated molecular active spaces is provided. The method includes receiving as input, for a chemical entity, (a) a geometry of the chemical entity, (b) at least one basis set for the chemical entity, and (c) a size of an active space for the chemical entity. For a plurality of N active molecular orbitals (MOs) and a plurality of M active electrons, the size of the active space is represented as CAS(Me, No). An active space ($A=\{m_1,m_2,m_3... m_N\}$) is formed as a sub-set of a set of MOs of the chemical entity relevant for one or more quantum calculations. The method further includes generating the set of MOs for the chemical entity by performing a Hartree-Fock calculation. The Hartree-Fock calculation linearly combines a plurality of atomic orbitals of one or more atoms in the chemical entity within the at least one basis set to form the set of MOs for the chemical entity. The method further includes calculating a threshold factor for the chemical entity. The threshold factor is defined as one angstrom added to one or more equilibrium bond distances between one or more individual atoms of the chemical entity wherein the addition of one to the equilibrium forms a distance after which a static correlation is assumed. The method further includes calculating an approximate ground state wavefunction specified in terms of the set of MOs, based on the threshold factor. The ground state wave function is calculated using one of a Coupled Cluster Singles and Doubles (CCSD) method when the input geometry of the chemical entity is lower than the threshold factor, and a Full Configuration Interaction (FCI) method when the input geometry of the chemical entity is higher than the threshold factor. The method further includes separately generating, a set SA of a plurality of active spaces ($A = \{m1,m2...mN\} \in SA$), of size CAS(Me, No) received for the chemical entity of size CAS(Me, No). The method further includes computing a correlation factor for each active space of the plurality of the active spaces of the size CAS(Me, No). The approximate ground state wavefunction calculated on the basis of the threshold factor is utilized in computing the correlation factor. The method further includes identifying, a sub-set of active spaces from the set SA of the plurality of active spaces by segregating the plurality of active spaces based on the correlation factor.

In another aspect, a system for selecting correlated molecular active spaces is provided. The system includes at least one memory storing programmed instructions; one or more Input /Output (I/O) interfaces; and one or more hardware processors, and an active space identification module operatively coupled to a corresponding at least one memory, wherein the system is configured to receive as input, for a chemical entity, (a) a geometry of the chemical entity, (b) at least one basis set for the chemical entity, and (c) a size of an active space for the chemical entity. For a plurality of N active molecular orbitals (MOs) and a plurality of M active electrons, the size of the active space is represented as CAS(Me, No). An active space ($A=\{m_1,m_2,m_3...m_N\}$) is formed as a sub-set of a set of MOs of the chemical entity relevant for one or more quantum calculations. Further the system is configured to generate the set of MOs for the chemical entity by performing a Hartree-Fock calculation. The Hartree-Fock calculation linearly combines a plurality of atomic orbitals of one or more atoms in the chemical entity within the at least one basis set to form the set of MOs for the chemical entity. Further the system is configured to calculate a threshold factor for the chemical entity. The threshold factor is defined as one angstrom added to one or more equilibrium bond distances between one or more individual atoms of the chemical entity wherein the addition of one to the equilibrium forms a distance after which a static correlation is assumed. Further the system is configured to calculate an approximate ground state wavefunction specified in terms of the set of MOs, based on the threshold factor. The ground state wave function is calculated using one of a Coupled Cluster Singles and Doubles (CCSD) method when the input geometry of the chemical entity is lower than the threshold factor, and a Full Configuration Interaction (FCI) method when the input geometry of the chemical entity is higher than the threshold factor. Further the system is configured to separately generate, a set SA of a plurality of active spaces ($A = \{m1,m2...mN\} \in SA$), of size CAS(Me, No) received for the chemical entity of size CAS(Me, No). Further the system is configured to compute a correlation factor for each active space of the plurality of the active spaces of the size CAS(Me, No). The approximate ground state wavefunction calculated on the basis of the threshold factor is utilized in computing the correlation factor. Further the system is configured to identify, a sub-set of active spaces from the set SA of the plurality of active spaces by segregating the plurality of active spaces based on the correlation factor.

In yet another aspect, a computer program product including a non-transitory computer-readable medium embodied therein a computer program for selecting correlated molecular active spaces is provided. The computer readable program, when executed on a computing device, causes the computing device to receive as input, for a chemical entity, (a) a geometry of the chemical entity, (b) at least one basis set for the chemical entity, and (c) a size of an active space for the chemical entity. For a plurality of N active molecular orbitals (MOs) and a plurality of M active electrons, the size of the active space is represented as CAS(Me, No). An active space ($A=\{m_1,m_2,m_3...m_N\}$) is formed as a sub-set of a set of MOs of the chemical entity relevant for one or more quantum calculations. The computer readable program, when executed on a computing device, causes the computing device to generate the set of MOs for the chemical entity by performing a Hartree-Fock calculation. The Hartree-Fock calculation linearly combines a plurality of atomic orbitals of one or more atoms in the chemical entity within the at least one basis set to form the set of MOs for the chemical entity. The computer readable program, when executed on a computing device, causes the computing device to calculate a threshold factor for the chemical entity. The threshold factor is defined as one angstrom added to one or more equilibrium bond distances between one or more individual atoms of the chemical entity wherein the addition of one to the equilibrium forms a distance after which a static correlation is assumed. The computer readable program, when executed on a computing device, causes the computing device to calculate an approximate ground state wavefunction specified in terms of the set of MOs, based on the threshold factor. The ground state wave function is calculated using one of a Coupled Cluster Singles and Doubles (CCSD) method when the input geometry of the chemical entity is lower than the threshold factor, and a Full Configuration Interaction (FCI) method when the input geometry of the chemical entity is higher than the threshold factor. The computer readable program, when executed on a computing device, causes the computing device to separately generate, a set SA of a plurality of active spaces ($A = \{m1,m2...mN\} \in SA$), of size CAS(Me, No) received for the chemical entity of size CAS(Me, No). The computer readable program, when executed on a computing device, causes the computing device to compute a correlation factor for each active space of the plurality of the active spaces of the size CAS(Me, No). The approximate ground state wavefunction calculated on the basis of the threshold factor is utilized in computing the correlation factor. The computer readable program, when executed on a computing device, causes the computing device to identify, a sub-set of active spaces from the set SA of the plurality of active spaces by segregating the plurality of active spaces based on the correlation factor.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system 100 for selection of correlated molecular active spaces, according to some embodiments of the present disclosure.

FIGS. 2A and 2B are flow diagrams of an illustrative method 200 for selection of correlated molecular active spaces, according to some embodiments of the present disclosure.

FIG. 3 is a functional flow diagram illustrating post Hartree-Fock methods (CCSD and FCI) of identifying an approximate ground state wavefunction, according to some embodiments of the present disclosure.

FIG. 4 is a visualization of the molecular orbitals (MOs) obtained from a restricted Hartree-Fock (RHF) calculation of water in STO-6G basis, used by the system of FIG. 1 during the selection of correlated molecular active spaces, according to some embodiments of the present disclosure.

FIG. 5 is a graph illustrating a symmetric dissociation profile obtained for H2O-CAS (6e,5o) dynamically selected active space, by the system of FIG. 1 during the selection of correlated molecular active spaces, according to some embodiments of the present disclosure.

FIG. 6 is a graph illustrating a CASCI energies for the chosen active spaces and the FCI energies for all the orbitals, by the system of FIG. 1 during the selection of correlated molecular active spaces, according to some embodiments of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following embodiments described herein.

As used herein the term 'atomic orbitals' or 'AOs' refers to a mathematical function describing the location and wave-like behavior of an electron in an atom.

As used herein the term 'molecular orbitals' or 'MOs' refers to a mathematical function describing the location and wave-like behavior of an electron in a molecule.

As used herein the term 'correlation factor', or 'CF' or 'E' are used interchangeably throughout the specification and refers to a mathematical quantity estimating the amount of electron correlation present in an active space.

As used herein the term 'active space' and 'molecular active space' are interchangeably used throughout the specification and refers to a set of molecular orbitals and electrons where interactions between one or more electrons is considered.

As used herein the term 'Input geometry' refers to a spatial arrangement of atoms in the chemical entity specified in terms of cartesian or internal coordinates.

In the present disclosure, identification of molecular active spaces with significant electron correlation energy begins post Hartree-Fock computation. A set of molecular orbitals are considered for identifying the active space containing significant correlation energy for a given molecular structure using any one of Coupled Cluster Singles and Doubles (CCSD) method or a Full Configuration Interaction (FCI) method. The CCSD method provides all possible single and double excitation amplitudes within the generated set of MOs in a given chemical entity at a chosen geometry and in a selected basis set. A reference dataset is formed that comprises of all possible single and double excitation amplitudes extracted from the CCSD calculation along with the indices of the molecular orbitals participating in each excitation. Next, all the possible active spaces for the specified number of active orbitals and electrons within the generated set of MOs are considered. Next, a correlation factor is computed for each active space (A) based on the possible single and double excitations within A and the corresponding excitation amplitudes derived from the reference dataset. It is estimated that the larger the correlation factor for an active space, the higher the degree of electron correlation present in it.

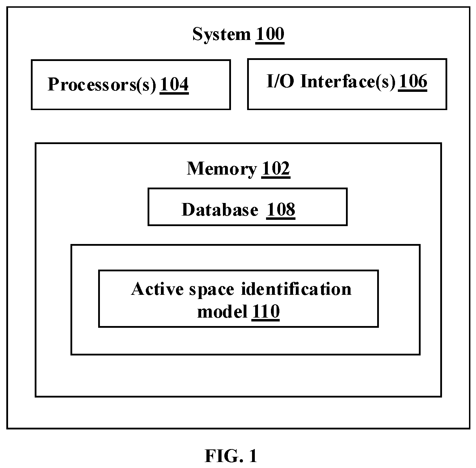

Referring now to the drawings, and more particularly to FIG. 1 through FIG. 6, where similar reference characters denote corresponding features consistently throughout the figures, there are shown preferred embodiments, and these embodiments are described in the context of the following exemplary system and/or method.

In an embodiment, the system 100 includes a processor(s) 104, communication interface device(s) 106, alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100. Referring to the components of system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, and the like. The I/O interface(s) 106 can include a variety of software and hardware interfaces, for example, a web interface, a graphical user interface to display the generated target images and the like and can facilitate multiple communications within a wide variety of networks N/W and protocol types, including wired networks, for example, LAN, cable, etc., and wireless networks, such as WLAN, cellular and the like. In an embodiment, the I/O interface (s) 106 can include one or more ports for connecting to number of external devices or to another server or devices. The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 102 includes an active space identification module 110. The active space identification module 110 considers a set of molecular orbitals (MOs) of a chemical entity that are obtained from Hartree-Fock calculations and identifies the active space within the chemical entity that are more relevant for quantum chemistry calculations based on a plurality of electron correlations among various electrons present in the active space. The model 110 performs the active space selection through highly accurate quantum chemistry methods like CCSD and FCI to identify both a static and a dynamic electron correlations. The modules, amongst other things, can include routines, programs, objects, components, and data structures, which perform particular tasks or implement particular abstract data types. The modules may also be used as signal processor(s), node machine(s), logic circuitries, and/or any other device or component that manipulates signals based on operational instructions. Further, the modules can be used by hardware, by computer-readable instructions executed by the one or more hardware processors 104, or by a combination thereof. The modules may include computer-readable instructions that supplement applications or functions performed by the system 100. Further, the memory 102 may comprise information pertaining to input(s)/output(s) of each step performed by the processor(s) 104 of the system 100 and methods of the present disclosure. Further, the memory 102 includes a database 108. The database (or repository) 108 may include a plurality of abstracted piece of code for refinement and data that is processed, received, or generated as a result of the execution of the plurality of modules. The external database is communicatively coupled to the system 100. The data contained within such an external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS).

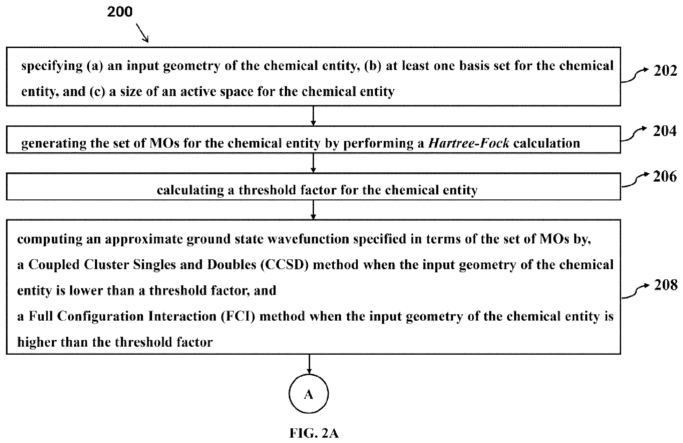

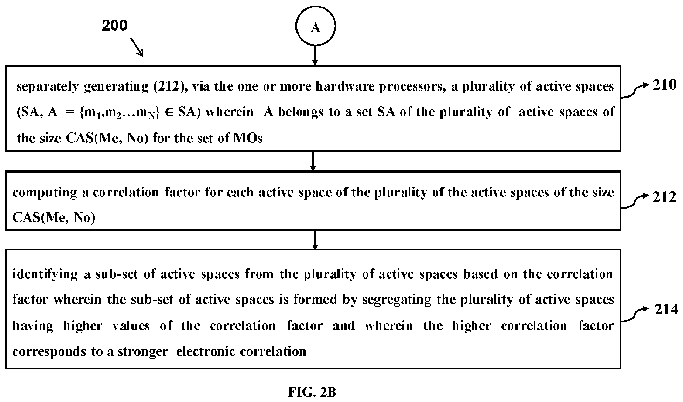

FIGS. 2A and 2B are flow diagrams of an illustrative method 200 for selection of correlated molecular active spaces, according to some embodiments of the present disclosure.

The steps of the method 200 of the present disclosure will now be explained with reference to the components of the system 100 as depicted in FIG. 1 through FIG. 6. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

At step 202 of the method 200, the one or more hardware processors 104 are configured to specify for a chemical entity (a) an input geometry of the chemical entity, (b) at least one basis set for the chemical entity, and (c) a size of an active space for the chemical entity. A user chooses the chemical entity of an interest wherein the user wishes to select an active space from the entire structure of the chemical entity which is more relevant for performing quantum chemistry calculations. The input geometry of the chemical entity comprises one or more bond lengths, one or more bond angles and one or more dihedral angles are specified of bond length of each bond between a plurality of atoms of the chemical entity. The bond length is expressed in angstrom and/or at various scales of angstrom. The input geometry comprises of a plurality of bond angles formed between neighboring atoms of the chemical entity. And the input geometry comprises of a plurality of dihedral angles depicting one or more molecular conformations formed between neighboring atoms of the chemical entity. The selection of an appropriate basis set for the chemical entity is important. The molecular spin-orbitals that are used in the Slater determinant usually are expressed as a linear combination of some chosen functions, which are called *basis functions.* This set of functions is called the *basis set.* The basis set is a set of functions (called basis functions) which are combined in linear combinations (generally as part of a quantum chemical calculation) to create molecular orbitals. Some of the commonly used basis set for quantum computation includes STO hierarchy basis sets, split-valence basis sets, pople basis sets, correlation-consistent basis sets, polarization-consistent basis sets, karlsruhe basis sets, completeness-optimized basis sets and even-tempered basis sets. In an embodiment, the STO hierarchy basis sets, STO-6G is used to model one or more chemical entities. The size of an active space for the chemical entity is specified by the user. The size of the active space is specified based on a computational efficiency of a system performing a plurality of quantum calculations to estimate an electron correlation. The active space is represented as a sub-set of the molecular orbitals of the chemical entity suitable for performing quantum computations based on favorable atomic arrangement and conformations. For a plurality of N active molecular orbitals (MOs) and a plurality of M active electrons represented as CAS(Me, No), the active space (A={$m_1,m_2,m_3...m_N$}) is formed as a subset of the MOs of the chemical entity relevant for one or more quantum chemistry calculations. At step 204 of the method 200, the one or more hardware processors 104 are configured to generate the set of MOs for the chemical entity by performing a Hartree-Fock calculation, wherein the Hartree-Fock calculation linearly combines a plurality of atomic orbitals of one or more atoms in the chemical entity within the at least one basis set to form the set of MOs for the chemical entity. The Hartree-Fock method is the basis of molecular orbital (MO) theory, which posits that each electron's motion can be described by a single-particle function (orbital) which does not depend explicitly on the instantaneous motions of the other electrons. The Hartree-Fock method consider an average (mean-field) contribution of the electron-electron interaction. The objective of Hartree-Fock (HF) theory is to produce the optimized MOs which are a combined form of contracted Cartesian Gaussian functions often referred to as Atomic Orbitals (AOs). The molecular orbitals are used to build the simplest possible antisymmetric wavefunction, a single Slater determinant. In the Hartree-Fock method the molecular orbitals thus obtained minimizes the energy of a system where electrons are treated as independent particles that experience a mean field generated by the other electrons. These optimized molecular orbitals are then used to construct one- and two- electron integrals in the basis of molecular orbitals.

At step 206 of the method 200, the one or more hardware processors 104 are configured to calculate a threshold factor for the chemical entity. The threshold factor is defined as one angstrom added to one or more equilibrium bond distances between one or more individual atoms of the chemical entity wherein the addition of one to the equilibrium forms a distance after which a static correlation is assumed.

At step 208 of the method 200, the one or more hardware processors 104 are configured to calculate an approximate ground state wavefunction specified in terms of the set of MOs, based on the threshold factor. The calculation of an approximate ground state wavefunction is performed by any one of a Coupled Cluster Singles and Doubles (CCSD) method or a Full Configuration Interaction (FCI) method. The CCSD method is selected when the input geometry of the chemical entity is lower than the threshold factor. And the FCI method is selected when the input geometry of the chemical entity is higher than the threshold factor. The true ground state wave function represents the lowest possible energy, so achieving the lower approximate energy, leads to the closer energy of the exact value. The CCSD method is a post-Hartree-Fock method capable of describing electron correlation in the ground state. It is size extensive but not variational. The standard approach for treating pair correlations self-consistently are CCSD methods where the cluster operator contains all single and double substitutions. The FCI method is a linear variational approach which provides numerically exact solutions (within the infinitely flexible complete basis set) to the electronic time-independent, non-relativistic Schrödinger equation.

At step 210 of the method 200, the one or more hardware processors 104 are configured to separately generate a set SA of a plurality of active spaces (A = {$m_1,m_2...m_N$} $\in$ SA), of size CAS(Me, No) received for the chemical entity of size CAS(Me, No). Once the ground state wave function for the generated MOs are obtained from either CCSD method or FCI method, all the possible active spaces are generated separately. To choose the number of MOs and electrons to be included in the active space CAS(Me, No), all the possible combinations of CAS(Me, No) active spaces that can be formed with all the available orbitals are to be considered. The choice of MOs in the active space for the chemical entity is done so that the static or non-dynamical correlation is qualitatively recovered in the wavefunction.

At step 212 of the method 200, the one or more hardware processors 104 are configured to compute a correlation factor for each active space of the plurality of the active spaces of the size CAS(Me, No), wherein the approximate ground state wavefunction calculated on the basis of the threshold factor is utilized in computing the correlation factor. When the CCSD method is adopted to calculate ground state wave function based on the threshold factor, the computation of the correlation factor involves consideration of all possible single and double excitation amplitudes.

A plurality of single excitation amplitudes ($t_i^a$), a plurality of double excitation amplitudes ($t_{ij}^{ab}$), and (c) a plurality of indices $i,j, ..$ (a,b,..) of one or more occupied orbitals and one or more virtual orbitals associated with each single and double excitation are first calculated. So, in the set of generated MOs, the plurality of single excitation amplitudes ($t_i^a$), the plurality of double excitation amplitudes ($t_{ij}^{ab}$), and the plurality of indices $i,j,..(a,b,..)$ of one or more occupied orbitals and one or more virtual orbitals associated with each single and double excitation wherein, the plurality of ($t_i^a$) and the plurality of indices (i, a) form a set S, and the plurality of ($t_{ij}^{ab}$) and the plurality of indices (i,j,a,b) form a set D. And the correlation factor ($\varepsilon$) for each active space (A) of the plurality of the active spaces (SA), based on the plurality of single excitation amplitudes ($t_i^a$), the plurality of double excitation amplitudes ($t_{ij}^{ab}$), and the plurality of indices $i,j,..(a,b,..)$ of one or more occupied orbitals and one or more virtual orbitals is calculated as:

$$\varepsilon = \sum_{t_i^a \in S, t_{ij}^{ab} \in D} |t_i^a|^2 + \left|t_{ij}^{ab}\right|^2 \qquad (1)$$

where $t_i^a$ is obtained from the Set S, and represents the amplitudes corresponding to the plurality of single excitations in the active space A, and where $t_{ij}^{ab}$ is obtained from the Set D, and represents the amplitudes corresponding to the plurality of double excitations in the active space A. The set S and the set D together form a reference dataset for the CCSD method. The reference dataset for the CCSD method comprises the plurality of single and double excitation amplitudes and the corresponding indices of the MOs and is utilized in calculating the correlation factor. Similarly, the ground state wavefunction obtained from the FCI method are utilized in calculating the correlation factor. Firstly, in a set of generated MOs, a plurality of excitation amplitudes of various orders are calculated. The plurality of excitation amplitudes further combines to calculate the correlation factor. In the generated set of MOs, (a) a plurality of excitation amplitudes of various orders corresponding to single excitation as $\left(t_{i_1}^{a_1}\right)$, double excitation as $\left(t_{i_1 i_2}^{a_1 a_2}\right)$ ......... upto $k^{th}$ order excitation as $\left(t_{i_1 i_2 .. i_k}^{a_1 a_2 .. a_k}\right)$, and (b) a plurality of indices $i_1, i_2,.. (a_1, a_2,..)$ of one or more occupied orbitals and one or more virtual orbitals corresponding to each single, double,... upto $k^{th}$ order excitation amplitudes are taken wherein the plurality of $t_{i_1}^{a_1}$ and indices $(i_1, a_1)$ forms a set $E_1$, the plurality of $\left(t_{i_1 i_2}^{a_1 a_2}\right)$ and indices $(i_1, i_2, a_1, a_2)$ forms a set $E_2$; and the plurality of $\left(t_{i_1 i_2 .. i_k}^{a_1 a_2 .. a_k}\right)$ and indices $(i_1, i_2 ... i_k, a_1, a_2,.. a_k)$ forms a set $E_k$. And the correlation factor $(\varepsilon)$ for each active space (A) of the plurality of the active spaces (SA) based on the plurality of excitation amplitudes of various orders and the plurality of indices $i_1, i_2,.. (a_1, a_2,..)$ of the one or more occupied orbitals and one or more virtual orbitals is calculated as:

$$\varepsilon = \sum_{t_{i_1}^{a_1} \in E_1, t_{i_1 i_2}^{a_1 a_2} \in E_2, ..., t_{i_1 i_2 .. i_k}^{a_1 a_2 .. a_k} \in E_k} \left|t_{i_1}^{a_1}\right|^2 + \left|t_{i_1 i_2}^{a_1 a_2}\right|^2 + .. + \left|t_{i_1 i_2 .. i_k}^{a_1 a_2 .. a_k}\right| \qquad (2)$$

where, $t_{i_1}^{a_1}$ is obtained from the Set $E_1$, and represents the amplitudes corresponding to the plurality of single excitations in the active space A, $t_{i_1 i_2}^{a_1 a_2}$ is obtained from the Set $E_2$, and represents the amplitudes corresponding to the plurality of double excitations in the active space A, and $t_{i_1 i_2 .. i_k}^{a_1 a_2 .. a_k}$ is obtained from the Set $E_k$, and represents the amplitudes corresponding to the plurality of $k^{th}$ order excitations in the active space A. The plurality of sets as set $E_1, E_2...$ upto $E_k$ together form a reference dataset for the FCI method. The reference dataset for the FCI method comprises the plurality of excitation amplitudes of various orders and the corresponding indices of the MOs and is utilized in calculating the correlation factor.

At step 214 of the method 200, the one or more hardware processors 104 are configured to identify a sub-set of active spaces from the plurality of active spaces SA based on the correlation factor wherein the sub-set of active spaces is formed by segregating the plurality of active spaces having higher values of the correlation factor. From the set of all the active spaces calculated separately, the correlation factor is computed for all the active spaces. A sub-set of active spaces is identified having higher correlation factor. The higher correlation factor corresponds to a stronger electronic correlation and hence is selected as the preferred active space. Thus, the sub-set of active spaces identified as per the present disclosure satisfies the active space size constraint specified by the user and at the same time recovers the electron correlation energy significantly.

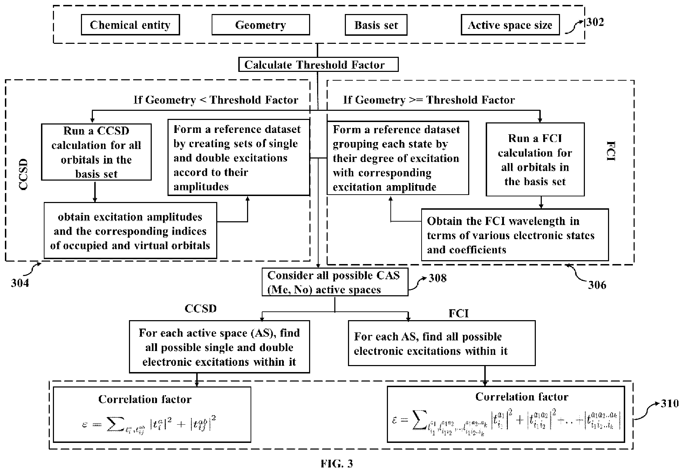

FIG. 3 is a functional flow diagram illustrating post Hartree-Fock methods (CCSD and FCI) of identifying an approximate ground state wavefunction, according to some embodiments of the present disclosure.

As illustrated in FIG. 3, the post Hartree-Fock computations are performed on the generated set of MOs to compute the correlation factor by one or more post Hartree-Fock methods, (i) the CCSD method, and (ii) the FCI method adopts. Both the methods adopt different methodology in managing the input. However, both methods have few convergences in terms of processing steps. At 302, the system 100 utilizes the input in terms of (a) chemical entity, (b) input geometry of the chemical entity, (c) basis set selected to process the input geometry, and (d) an active space size specified by the user, the correlation factor is computed by following two different routes, (i) the CCSD method, and (ii) the FCI method. In the first route at 304, the CCSD calculations are performed by including all the orbitals in the chosen basis set for the specified geometry. Further, all the single (._.^.) and double (._..^..) excitation amplitudes and the indices .,.,..(.,.,..) of the occupied (virtual) orbitals corresponding to each single and double excitation are obtained. The single and double excitations aid in identifying the dominant electronic excitations and can serve as reference datasets for choosing an active space with high correlation energy. The number of molecular orbitals are chosen to be included in the active space are represented as CAS(Me, No). Similarly, in the second route at 306, the FCI calculations are performed by including all the orbitals in the chosen basis set for the specified geometry. The FCI ground state wavefunction is obtained in terms of various electronic states and coefficients. Then a reference dataset is formed by grouping each state by their degree of excitation with the corresponding coefficient. These reference dataset is calculated once for the plurality of MOs and is referred to identify the plurality of active spaces based on the user preference to perform the quantum calculations. At 308, all possible combinations of CAS(Me, No) active spaces that can be formed with all the available orbitals are considered. Finally, at 310, for each active space, the correlation factor is computed.

## USE CASE

An example scenario of selecting an active space with maximum correlation energy in a chemical entity such as water molecule ($H_2O$) by the disclosed method utilizing the system 100 is presented below. A minimal basis set, STO-6G is chosen to model $H_2O$ molecule resulting in a total of 7 orbitals. Employing the minimal basis set found suitable to perform CCSD calculations smoothly on a classical computer.

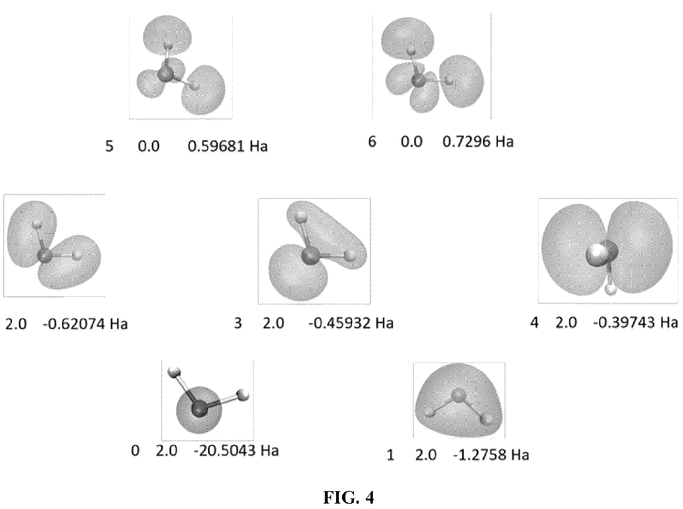

For $H_2O$, the interest is to compute the symmetric dissociation profile, which shows the variation of the ground state potential energy as a function of oxygen-hydrogen ($R_{OH}$) distances at a fixed H-O-H angle. This is a typical case of strongly-correlated problem where conventional CCSD tends to overestimate the energies, specifically at large ROH values. The one- and two-electron integrals defining the electronic Hamiltonian are computed in the canonical restricted HF molecular orbital basis using the PySCF package. The same package is used to run the classical electronic structure methods FCI, CCSD, CASCI, which are used to benchmark the quantum computing approach of the present disclosure. The H-O-H angle of $H_2O$ is fixed at the experimental equilibrium value of 104.478° and the two hydrogen atoms are symmetrically displaced away from the oxygen atom from 0.5 Å to 2.4 Å. For each $R_{OH}$ distance, a restricted HF (RHF) calculation is performed in the STO-6G basis, giving rise to seven molecular orbitals as depicted in FIG. 4 for $R_{OH}$ = 0.958 Å. The active space of five spatial orbitals and six electrons, CAS(6e,5o) is considered, for all bond distances, and to determine the orbitals that recover the maximum correlation energy. The approach of selecting the active space containing significant correlation energy for a given molecular structure includes (i) run a CCSD calculation including all the orbitals in the chosen basis set for the specified geometry; and (ii) obtain all the single $t_i^a$ and double $t_{ij}^{ab}$ excitation amplitudes and the indices i, j,.. (a,b,..) of the occupied (virtual) orbitals corresponding to each single and double excitation. These sets of single and double excitations, denoted as S and D, respectively, assist in identifying the dominant electronic excitations (ones with larger amplitudes). This serves as reference datasets for choosing an active space with high correlation energy. Finally, all possible combinations of CAS(Me, No) active spaces that can be formed with all the available orbitals are considered and for each active space the correlation factor $\varepsilon$ is computed as:

$$\varepsilon = \sum_{t_i^a, t_{i,j}^{a,b}} |t_i^a|^2 + |t_{ij}^{ab}|^2 \qquad (3)$$

where $t_i^a \in S$ and $t_{ij}^{ab} \in D$ are the amplitudes corresponding to all the possible single and double excitations in the chosen active space. It is concluded that the larger the correlation factor of an active space, the stronger the amount of electron correlation present.

For the equilibrium ($R_{OH}$) value of 0.958Å, the different choices of CAS(6e,5o) active spaces, their associated correlation factor, and the correlation energy calculated with CCSD are given in Table-1. The correlation factor $\varepsilon$, total ground state energy EAS-CCSD in Hartrees (Ha), and correlation energy in milli-Hartrees (mHa) computed using CCSD for each active space are also given.

Table-1

| Method | Frozen Orbitals | Active Orbitals | CAS(Me, No) | $\varepsilon$ | Total GSE (Ha) | Correlation Energy (mHa) | Absolute energy error w.r.t full CCSD (mHa) |
|---|---|---|---|---|---|---|---|
| CCSD | [0,4] | (1, 2, 3, 5, 6) | (6e, 5o) | 0.0257933 | -75.727687 | -48.8990 | 1.100 |
| CCSD | [0,1] | (2, 3, 4, 5, 6) | (6e, 5o) | 0.0201198 | -75.71287 | -34.0843 | 15.915 |
| CCSD | [1,4] | (0, 2, 3, 5, 6) | (6e, 5o) | 0.0193250 | -75.711727 | -32.9384 | 17.061 |
| CCSD | [0,3] | (1, 2, 4, 5, 6) | (6e, 5o) | 0.0131783 | -75.707899 | -29.1106 | 20.88 |
| CCSD | [3,4] | (0, 1, 2, 5, 6) | (6e, 5o) | 0.0123842 | - 75.7068796 | -28.0907 | 21.908 |
| CCSD | [1,3] | (0, 2, 4, 5, 6) | (6e, 5o) | 0.008966 | - 75.6955068 | -16.7179 | 33.281 |
| CCSD | [0,2] | (1, 3, 4, 5, 6) | (6e, 5o) | 0.008543 | - 75.6950993 | -16.3104 | 33.689 |
| CCSD | [2,4] | (0, 1, 3, 5, 6) | (6e, 5o) | 0.0077499 | - 75.6940200 | -15.231 | 34.7683 |
| CCSD | [1,2] | (0, 3, 4, 5, 6) | (6e, 5o) | 0.0049414 | - 75.6862987 | -7.50987 | 42.4896 |
| CCSD | [2,3] | (0, 1, 4, 5, 6) | (6e, 5o) | 0.0021449 | - 75.6849409 | -6.15200 | 43.8474 |

It is evident from Table-1 that active spaces with higher correlation energy have a larger correlation factor associated with them. This implies that the correlation factor serves as a good metric to determine the orbitals that retain significant correlation energy. The main computational difficulty in applying this method is the requirement of running a CCSD calculation including all the orbitals in a chosen basis set, which has a polynomial scaling with the system size (number of basis functions). The method disclosed in the present invention was found to be particularly useful in identifying active spaces with good correlation. By dynamically varying the set of active orbitals depending on the highest correlation factor determines the best active space at each ROH value. The range of ROH values and the corresponding choice of active orbitals are given in Table-2.

Table-2

| $R_{OH}$ (Å) | Active orbitals |
|---|---|
| <1.4 | (1,2,3,5,6) |
| [1.4, 1.5] | (1,2,4,5,6) |
| >1.5 | (1,3,4,5,6) |

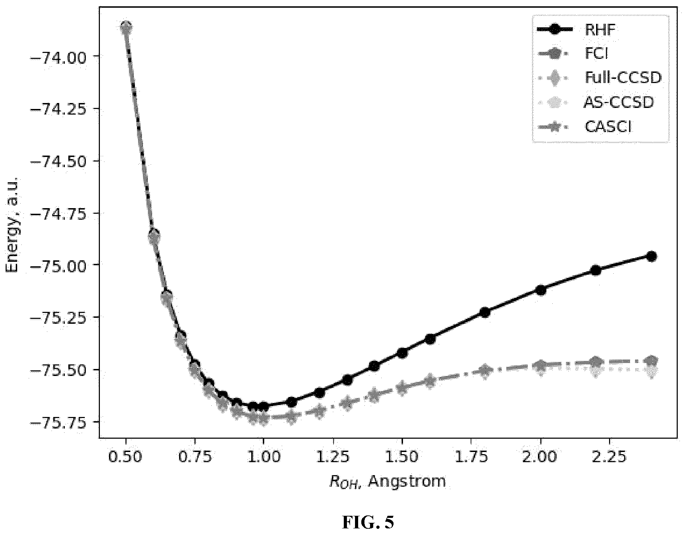

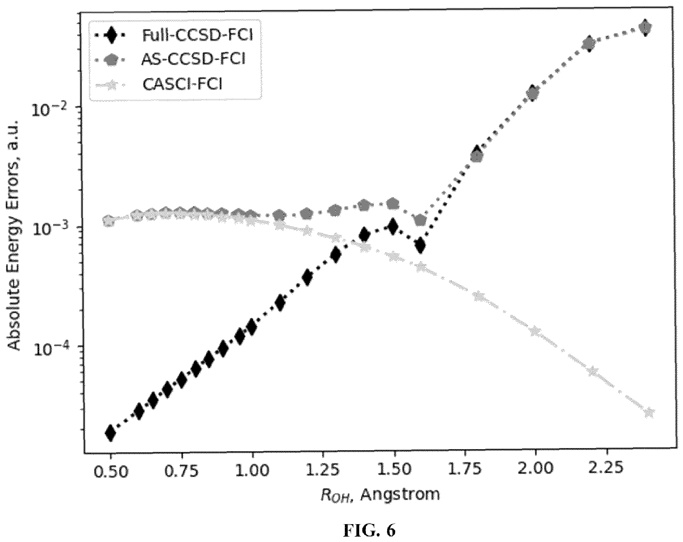

The dissociation profiles obtained with various electronic structure methods are shown in FIG. 5. One of the main observations is that the chosen active space, CAS(6e, 5o) (given in Table-2) is able to recover almost all the correlation energy for the entire range of bond distances, leading to a perfectly accurate description of the O-H bond dissociation. To quantify this, the absolute energy difference is plotted between the CASCI energies for the chosen active spaces and the FCI energies for all the orbitals as shown in FIG. 6. The energy errors varied from a few milli-Hartrees at smaller bond lengths to hundreds of micro-Hartrees at large distances. Secondly, both CCSD calculations including all the orbitals (Full-CCSD) and in the active space (AS-CCSD) produced large deviations from the FCI energies near the bond dissociation limit. Therefore, the method disclosed in the present invention accurately performed the active space selection.

The written description describes the subject matter herein to enable any person skilled in the art to make and use the embodiments. The scope of the subject matter embodiments is defined herein and may include other modifications that occur to those skilled in the art. Such other modifications are intended to be within the scope of the present disclosure if they have similar elements that do not differ from the literal language of the embodiments or if they include equivalent elements with insubstantial differences from the literal language of the embodiments described herein.

Therefore, the method disclosed in the present invention effectively determine the dominant electronic excitations in a given molecule at a chosen geometry and in a selected basis set utilizing the highly accurate CCSD and FCI methods. The reference dataset is formed comprising of all possible single and double excitation amplitudes extracted from the CCSD and FCI methods respectively which are utilized in calculating and identifying the molecular orbitals participating in each excitation. The disclosed method consider all possible choices of active spaces for a specified number of active orbitals and electrons and compute a quantity called correlation factor for each active space based on the possible excitations within it and the previously stored excitation amplitudes. It is concluded that the larger the correlation factor for an active space, the stronger the degree of electron correlation present in it.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (200) of selecting correlated molecular active spaces, the method comprising:

   receiving as input (202), for a chemical entity, via one or more hardware processors,

   (a) a geometry of the chemical entity,
   (b) at least one basis set for the chemical entity, and
   (c) a size of an active space for the chemical entity, wherein for a plurality of N active molecular orbitals (MOs) and a plurality of M active electrons, the size of the active space is represented as CAS(Me, No),

   and wherein an active space $(A=\{m_1,m_2,m_3...m_N\})$ is formed as a sub-set of a set of MOs of the chemical entity relevant for one or more quantum calculations;
   generating (204), via the one or more hardware processors, the set of MOs for the chemical entity by performing a Hartree-Fock calculation, wherein the Hartree-Fock calculation linearly combines a plurality of atomic orbitals of one or more atoms in the chemical entity within the at least one basis set to form the set of MOs for the chemical entity;

calculating (206), via the one or more hardware processors, a threshold factor for the chemical entity;

calculating (208), via the one or more hardware processors, an approximate ground state wavefunction specified in terms of the set of MOs, based on the threshold factor;

separately generating (210), via the one or more hardware processors, a set SA of a plurality of active spaces (A = {m1,m2...mN} ∈ SA), of size CAS(Me, No) received for the chemical entity of size CAS(Me, No);

computing (212), via the one or more hardware processors, a correlation factor for each active space of the plurality of the active spaces of the size CAS(Me, No), wherein the approximate ground state wavefunction calculated on the basis of the threshold factor is utilized in computing the correlation factor; and

identifying (214), via the one or more hardware processors, a sub-set of active spaces from the set SA of the plurality of active spaces by segregating the plurality of active spaces based on the correlation factor.

2. The method as claimed in claim 1, wherein the size of the active space is specified based on a computational efficiency of a system performing a plurality of quantum calculations to estimate an electron correlation.

3. The method as claimed in claim 1, wherein the approximate ground state wavefunction is calculated based on the threshold factor by,

a Coupled Cluster Singles and Doubles (CCSD) method when the input geometry of the chemical entity is lower than the threshold factor, and a Full Configuration Interaction (FCI) method when the input geometry of the chemical entity is higher than the threshold factor.

4. The method as claimed in claim 3, wherein the approximate ground state wavefunction obtained from the CCSD method is utilized in calculating the correlation factor, by:

calculating, in the generated set of MOs, (a) a plurality of single excitation amplitudes ( $t_i^a$ ), (b) a plurality of double excitation amplitudes ( $t_{ij}^{ab}$ ), and (c) a plurality of indices i, j,.. (a,b,..) of one or more occupied orbitals and one or more virtual orbitals associated with each single and double excitation wherein the plurality of ( $t_i^a$ ) and the plurality of indices (i, a) form a set S, and the plurality of ( $t_{ij}^{ab}$ ) and the plurality of indices (i,j,a,b) form a set D; and computing the correlation factor (ε) for each active space (A) of the plurality of the active spaces (SA), based on the plurality of single excitation amplitudes ( $t_i^a$ ), the plurality of double excitation amplitudes ( $t_{ij}^{ab}$ ), and the plurality of indices i,j,..(a,b,..) of one or more occupied orbitals and one or more virtual orbitals as:

$$\varepsilon = \sum_{t_i^a \in S, t_{ij}^{ab} \in D} |t_i^a|^2 + \left|t_{ij}^{ab}\right|^2$$

where $t_i^a$ is obtained from the Set S, and represents the amplitudes corresponding to the plurality of single excitations in the active space A, and where $t_{ij}^{ab}$ is obtained from the Set D, and represents the amplitudes corresponding to the plurality of double excitations in the active space A.

5. The method as claimed in claim 3, wherein the ground state wavefunction obtained from the FCI method are utilized in calculating the correlation factor by:

calculating, in the generated set of MOs, (a) a plurality of excitation amplitudes of various orders corresponding to single excitation as $\left(t_{i_1}^{a_1}\right)$, double excitation as $\left(t_{i_1 i_2}^{a_1 a_2}\right)$,........ upto k$^{th}$ order excitation as $\left(t_{i_1 i_2..i_k}^{a_1 a_2..a_k}\right)$, and (b) a plurality of indices $i_1, i_2, .. (a_1, a_2,..)$ of one or more occupied orbitals and one or more virtual orbitals corresponding to each single, double,... upto $k^{th}$ order excitation amplitudes wherein the plurality of $t_{i_1}^{a_1}$ and indices $(i_1, a_1)$ forms a set $E_1$, the plurality of $\left(t_{i_1 i_2}^{a_1 a_2}\right)$ and indices $(i_1, i_2, a_1, a_2)$ forms a set $E_2$; and the plurality of $\left(t_{i_1 i_2..i_k}^{a_1 a_2..a_k}\right)$ and indices $(i_1, i_2...i_k, a_1, a_2,.. a_k)$ forms a set $E_k$; and

computing the correlation factor $(\varepsilon)$ for each active space (A) of the plurality of the active spaces (SA) based on the plurality of excitation amplitudes of various orders and the plurality of indices $i_1, i_2,.. (a_1, a_2,..)$ of the one or more occupied orbitals and one or more virtual orbitals as:

$$\varepsilon = \sum_{t_{i_1}^{a_1} \in E_1, t_{i_1 i_2}^{a_1 a_2} \in E_2,.., t_{i_1 i_2..i_k}^{a_1 a_2..a_k} \in E_k} \left|t_{i_1}^{a_1}\right|^2 + \left|t_{i_1 i_2}^{a_1 a_2}\right|^2 +.. + \left|t_{i_1 i_2..i_k}^{a_1 a_2..a_k}\right|$$

where, $t_{i_1}^{a_1}$ is obtained from the Set $E_1$, and represents the amplitudes corresponding to the plurality of single excitations in the active space A, $t_{i_1 i_2}^{a_1 a_2}$ is obtained from the Set $E_2$, and represents the amplitudes corresponding to the plurality of double excitations in the active space A, and $t_{i_1 i_2..i_k}^{a_1 a_2..a_k}$ is obtained from the Set $E_k$, and represents the amplitudes corresponding to the plurality of $k^{th}$ order excitations in the active space A.

6. The method as claimed in claim 4, wherein the set S and the set D together form a reference dataset for the CCSD method, and wherein the reference dataset for the CCSD method comprises the plurality of single and double excitation amplitudes and the corresponding indices of the MOs and is utilized in calculating the correlation factor.

7. The method as claimed in claim 5, wherein the plurality of sets as set $E_1$, $E_2$...
upto $E_k$ together form a reference dataset for the FCI method, and wherein the reference dataset for the FCI method comprises the plurality of excitation amplitudes of various orders and the corresponding indices of the MOs and is utilized in calculating the correlation factor.

8. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

receive, as input, for a chemical entity,

(a) a geometry of the chemical entity,
(b) at least one basis set for the chemical entity, and
(c) a size of an active space for the chemical entity, wherein for a plurality of N active molecular orbitals (MOs) and a plurality of M active electrons, the size of the active space is represented as CAS(Me, No),

and wherein an active space $(A=\{m_1, m_2, m_3...m_N\})$ is formed as a sub-set of a set of MOs of the chemical entity relevant for one or more quantum calculations;
generate the set of MOs for the chemical entity by performing a Hartree-Fock calculation, wherein the Hartree-Fock calculation linearly combines a plurality of atomic orbitals of one or more atoms in the chemical entity within the at least one basis set to form the set of MOs for the chemical entity;
calculate a threshold factor for the chemical entity;
calculate an approximate ground state wavefunction specified in terms of the set of MOs, based on the threshold factor;
separately generate a set SA of a plurality of active spaces $(A = \{m1, m2...mN\} \in SA)$, of size CAS(Me, No) received for the chemical entity of size CAS(Me, No);

compute a correlation factor for each active space of the plurality of the active spaces of the size CAS(Me, No), wherein the approximate ground state wavefunction calculated on the basis of the threshold factor is utilized in computing the correlation factor; and

identify a sub-set of active spaces from the set SA of the plurality of active spaces by segregating the plurality of active spaces based on the correlation factor.

9. The system as claimed in claim 8, wherein the size of the active space is specified based on a computational efficiency of a system performing a plurality of quantum calculations to estimate an electron correlation.

10. The system as claimed in claim 8, wherein the approximate ground state wavefunction is calculated based on the threshold factor by,

a Coupled Cluster Singles and Doubles (CCSD) method when the input geometry of the chemical entity is lower than the threshold factor, and a Full Configuration Interaction (FCI) method when the input geometry of the chemical entity is higher than the threshold factor.

11. The system as claimed in claim 10, wherein the approximate ground state wavefunction obtained from the CCSD method is utilized in calculating the correlation factor, by:

calculating, in the generated set of MOs, (a) a plurality of single excitation amplitudes ( $t_i^a$ ), (b) a plurality of double excitation amplitudes ( $t_{ij}^{ab}$ ), and (c) a plurality of indices $i,j,..(a,b,..)$ of one or more occupied orbitals and one or more virtual orbitals associated with each single and double excitation wherein the plurality of ( $t_i^a$ ) and the plurality of indices (i, a) form a set S, and the plurality of ( $t_{ij}^{ab}$ ) and the plurality of indices (i,j,a,b) form a set D; and computing the correlation factor ($\varepsilon$) for each active space (A) of the plurality of the active spaces (SA), based on the plurality of single excitation amplitudes ( $t_i^a$ ), the plurality of double excitation amplitudes ( $t_{ij}^{ab}$ ), and the plurality of indices $i,j,..(a,b,..)$ of one or more occupied orbitals and one or more virtual orbitals as:

$$\varepsilon = \sum_{t_i^a \in S, t_{ij}^{ab} \in D} |t_i^a|^2 + \left|t_{ij}^{ab}\right|^2$$

where $t_i^a$ is obtained from the Set S, and represents the amplitudes corresponding to the plurality of single excitations in the active space A, and where $t_{ij}^{ab}$ is obtained from the Set D, and represents the amplitudes corresponding to the plurality of double excitations in the active space A.

12. The system as claimed in claim 10, wherein the ground state wavefunction obtained from the FCI method are utilized in calculating the correlation factor by:

calculating, in the generated set of MOs, (a) a plurality of excitation amplitudes of various orders corresponding to single excitation as $\left(t_{i_1}^{a_1}\right)$, double excitation as $\left(t_{i_1 i_2}^{a_1 a_2}\right)$,........ upto $k^{th}$ order excitation as $\left(t_{i_1 i_2..i_k}^{a_1 a_2..a_k}\right)$, and (b) a plurality of indices $i_1, i_2,..(a_1, a_2,..)$ of one or more occupied orbitals and one or more virtual orbitals corresponding to each single, double,... upto $k^{th}$ order excitation amplitudes wherein the plurality of $t_{i_1}^{a_1}$ and indices $(i_1, a_1)$ forms a set $E_1$, the plurality of $\left(t_{i_1 i_2}^{a_1 a_2}\right)$ and indices $(i_1, i_2, a_1, a_2)$ forms a set $E_2$; and the plurality of $\left(t_{i_1 i_2..i_k}^{a_1 a_2..a_k}\right)$ and indices $(i_1, i_2....i_k, a_1, a_2,.. a_k)$ forms a set $E_k$; and computing the correlation factor ($\varepsilon$) for each active space (A) of the plurality of the active spaces (SA) based on the plurality of excitation amplitudes of various orders and the plurality of indices $i_1, i_2,..$ $(a_1, a_2,..)$ of the one or more occupied orbitals and one or more virtual orbitals as:

$$\varepsilon = \sum_{t_{i_1}^{a_1} \in E_1, t_{i_1 i_2}^{a_1 a_2} \in E_2, \dots, t_{i_1 i_2 \dots i_k}^{a_1 a_2 \dots a_k} \in E_k} \left| t_{i_1}^{a_1} \right|^2 + \left| t_{i_1 i_2}^{a_1 a_2} \right|^2 + \dots + \left| t_{i_1 i_2 \dots i_k}^{a_1 a_2 \dots a_k} \right|$$

where, $t_{i_1}^{a_1}$ is obtained from the Set $E_1$, and represents the amplitudes corresponding to the plurality of single excitations in the active space A, $t_{i_1 i_2}^{a_1 a_2}$ is obtained from the Set $E_2$, and represents the amplitudes corresponding to the plurality of double excitations in the active space A, and $t_{i_1 i_2 \dots i_k}^{a_1 a_2 \dots a_k}$ is obtained from the Set $E_k$, and represents the amplitudes corresponding to the plurality of $k^{th}$ order excitations in the active space A.

13. The system as claimed in claim 11, wherein the set S and the set D together form a reference dataset for the CCSD method, and wherein the reference dataset for the CCSD method comprises the plurality of single and double excitation amplitudes and the corresponding indices of the MOs and is utilized in calculating the correlation factor.

14. The system as claimed in claim 12, wherein the plurality of sets as set $E_1, E_2...$ upto $E_k$ together form a reference dataset for the FCI method, and wherein the reference dataset for the FCI method comprises the plurality of excitation amplitudes of various orders and the corresponding indices of the MOs and is utilized in calculating the correlation factor.

15. One or more non-transitory machine-readable information storage mediums comprising one or more instructions which when executed by one or more hardware processors cause:

receiving as an input, for a chemical entity,

(a) a geometry of the chemical entity,
(b) at least one basis set for the chemical entity, and
(c) a size of an active space for the chemical entity, wherein for a plurality of N active molecular orbitals (MOs) and a plurality of M active electrons, the size of the active space is represented as CAS(Me, No),

and wherein an active space ($A=\{m_1, m_2, m_3...m_N\}$) is formed as a sub-set of a set of MOs of the chemical entity relevant for one or more quantum calculations;
generating the set of MOs for the chemical entity by performing a Hartree-Fock calculation, wherein the Hartree-Fock calculation linearly combines a plurality of atomic orbitals of one or more atoms in the chemical entity within the at least one basis set to form the set of MOs for the chemical entity;
calculating a threshold factor for the chemical entity;
calculating an approximate ground state wavefunction specified in terms of the set of MOs, based on the threshold factor;
separately generating, via the one or more hardware processors, a set SA of a plurality of active spaces (A = $\{m1, m2...mN\} \in$ SA), of size CAS(Me, No) received for the chemical entity of size CAS(Me, No);
computing a correlation factor for each active space of the plurality of the active spaces of the size CAS(Me, No), wherein the approximate ground state wavefunction calculated on the basis of the threshold factor is utilized in computing the correlation factor; and
identifying a sub-set of active spaces from the set SA of the plurality of active spaces by segregating the plurality of active spaces based on the correlation factor.

**System 100**

**Processors(s) 104**

**I/O Interface(s) 106**

**Memory 102**

**Database 108**

**Active space identification model 110**

**FIG. 1**

**200**

| specifying (a) an input geometry of the chemical entity, (b) at least one basis set for the chemical entity, and (c) a size of an active space for the chemical entity | 202 |

| generating the set of MOs for the chemical entity by performing a *Hartree-Fock* calculation | 204 |

| calculating a threshold factor for the chemical entity | 206 |

| computing an approximate ground state wavefunction specified in terms of the set of MOs by, a Coupled Cluster Singles and Doubles (CCSD) method when the input geometry of the chemical entity is lower than a threshold factor, and a Full Configuration Interaction (FCI) method when the input geometry of the chemical entity is higher than the threshold factor | 208 |

A

FIG. 2A

**200**

(A)

separately generating (212), via the one or more hardware processors, a plurality of active spaces (SA, A = {$m_1,m_2...m_N$} $\in$ SA) wherein A belongs to a set SA of the plurality of active spaces of the size CAS(Me, No) for the set of MOs

↗ **210**

computing a correlation factor for each active space of the plurality of the active spaces of the size CAS(Me, No)

↗ **212**

identifying a sub-set of active spaces from the plurality of active spaces based on the correlation factor wherein the sub-set of active spaces is formed by segregating the plurality of active spaces having higher values of the correlation factor and wherein the higher correlation factor corresponds to a stronger electronic correlation

↗ **214**

**FIG. 2B**

Chemical entity | Geometry | Basis set | Active space size | 302

Calculate Threshold Factor

If Geometry < Threshold Factor | If Geometry >= Threshold Factor

CCSD

Run a CCSD calculation for all orbitals in the basis set

Form a reference dataset by creating sets of single and double excitations accord to their amplitudes

Form a reference dataset grouping each state by their degree of excitation with corresponding excitation amplitude

Run a FCI calculation for all orbitals in the basis set

FCI

obtain excitation amplitudes and the corresponding indices of occupied and virtual orbitals

Obtain the FCI wavelength in terms of various electronic states and coefficients

304

Consider all possible CAS (Me, No) active spaces | 308

306

CCSD

For each active space (AS), find all possible single and double electronic excitations within it

FCI

For each AS, find all possible electronic excitations within it

Correlation factor

$$\varepsilon = \sum_{t_i^a, t_{ij}^{ab}} |t_i^a|^2 + |t_{ij}^{ab}|^2$$

Correlation factor

$$\varepsilon = \sum_{t_{i_1}^{a_1}, t_{i_1 i_2}^{a_1 a_2} \cdots t_{i_1 i_2 \cdots i_k}^{a_1 a_2 \cdots a_k}} |t_{i_1}^{a_1}|^2 + |t_{i_1 i_2}^{a_1 a_2}|^2 + \ldots + |t_{i_1 i_2 \cdots i_k}^{a_1 a_2 \cdots a_k}|$$

310

FIG. 3

5　0.0　0.59681 Ha

6　0.0　0.7296 Ha

2.0　-0.62074 Ha

3　2.0　-0.45932 Ha

4　2.0　-0.39743 Ha

0　2.0　-20.5043 Ha

1　2.0　-1.2758 Ha

**FIG. 4**

**FIG. 5**

**FIG. 6**

# EUROPEAN SEARCH REPORT

Europäisches
Patentamt

European
Patent Office

Office européen
des brevets

**Application Number**

EP 25 18 4363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KAUFOLD BENJAMIN W. ET AL: "Automated Active Space Selection with Dipole Moments", JOURNAL OF CHEMICAL THEORY AND COMPUTATION: JCTC, vol. 19, no. 9, 11 April 2023 (2023-04-11), pages 2469-2483, XP093341908, US ISSN: 1549-9618, DOI: 10.1021/acs.jctc.2c01128 Retrieved from the Internet: URL:https://pubs.acs.org/doi/pdf/10.1021/acs.jctc.2c01128> * the whole document * ----- | 1-15 | INV. G16C10/00 |
| X | STEIN CHRISTOPHER J. ET AL: "Automated Selection of Active Orbital Spaces", JOURNAL OF CHEMICAL THEORY AND COMPUTATION: JCTC, vol. 12, no. 4, 21 March 2016 (2016-03-21), pages 1760-1771, XP093341909, US ISSN: 1549-9618, DOI: 10.1021/acs.jctc.6b00156 * the whole document * ----- | 1-15 | TECHNICAL FIELDS SEARCHED (IPC) G16C |
| X | KHEDKAR ABHISHEK ET AL: "Active Space Selection Based on Natural Orbital Occupation Numbers from n -Electron Valence Perturbation Theory", JOURNAL OF CHEMICAL THEORY AND COMPUTATION: JCTC, vol. 15, no. 6, 6 May 2019 (2019-05-06), pages 3522-3536, XP093341910, US ISSN: 1549-9618, DOI: 10.1021/acs.jctc.8b01293 * the whole document * ----- -/-- | 1-15 | |

The present search report has been drawn up for all claims

| Place of search | Date of completion of the search | Examiner |
|---|---|---|
| The Hague | 1 December 2025 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**EP 4 687 144 A1**

# EUROPEAN SEARCH REPORT

**Application Number**

EP 25 18 4363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | IZSÁK RÓBERT ET AL: "Quantum computing in pharma: A multilayer embedding approach for near future applications", JOURNAL OF COMPUTATIONAL CHEMISTRY., vol. 44, no. 3, 5 July 2022 (2022-07-05), pages 406-421, XP093341906, GB ISSN: 0192-8651, DOI: 10.1002/jcc.26958 Retrieved from the Internet: URL:https://onlinelibrary.wiley.com/doi/full-xml/10.1002/jcc.26958> * the whole document * | 1-15 | |
| X | SCHEURER MAXIMILIAN ET AL: "Tailored and Externally Corrected Coupled Cluster with Quantum Inputs", JOURNAL OF CHEMICAL THEORY AND COMPUTATION: JCTC, vol. 20, no. 12, 3 June 2024 (2024-06-03), pages 5068-5093, XP093341907, US ISSN: 1549-9618, DOI: 10.1021/acs.jctc.4c00037 Retrieved from the Internet: URL:https://pubs.acs.org/doi/pdf/10.1021/acs.jctc.4c00037> * the whole document * | 1-15 | |
| X | EP 4 243 025 A1 (FUJITSU LTD [JP]) 13 September 2023 (2023-09-13) * the whole document * | 1-15 | **TECHNICAL FIELDS SEARCHED (IPC)** |
| | -/-- | | |

The present search report has been drawn up for all claims

| Place of search | Date of completion of the search | Examiner |
|---|---|---|
| The Hague | 1 December 2025 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 .............................................................................

& : member of the same patent family, corresponding document

EPO FORM 1503 03.82 (P04C01)

page 2 of 3

Europäisches
Patentamt

European
Patent Office

Office européen
des brevets

## EUROPEAN SEARCH REPORT

**Application Number**

EP 25 18 4363

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TIM WEAVING ET AL: "Contextual Subspace Variational Quantum Eigensolver Calculation of the Dissociation Curve of Molecular Nitrogen on a Superconducting Quantum Computer", ARXIV.ORG, CORNELL UNIVERSITY LIBRARY, 201 OLIN LIBRARY CORNELL UNIVERSITY ITHACA, NY 14853, 24 July 2024 (2024-07-24), XP091824951, * the whole document * | 1-15 | |

-----

TECHNICAL FIELDS
SEARCHED       (IPC)

The present search report has been drawn up for all claims

| Place of search | Date of completion of the search | Examiner |
|---|---|---|
| The Hague | 1 December 2025 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 ...........................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 4363

This annex lists the patent family members relating to the patent documents cited in the above-mentioned European search report.
The members are as contained in the European Patent Office EDP file on
The European Patent Office is in no way liable for these particulars which are merely given for the purpose of information.

01-12-2025

| Patent document cited in search report | | Publication date | Patent family member(s) | | Publication date |
|---|---|---|---|---|---|
| EP 4243025 | A1 | 13-09-2023 | CN | 116490928 A | 25-07-2023 |
| | | | EP | 4243025 A1 | 13-09-2023 |
| | | | JP | 7506330 B2 | 26-06-2024 |
| | | | JP | WO2022097298 A1 | 12-05-2022 |
| | | | US | 2024013865 A1 | 11-01-2024 |
| | | | WO | 2022097298 A1 | 12-05-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421058105